# EUROPEAN PATENT SPECIFICATION

(11) **EP 2 394 682 B1**
(45) Date of publication and mention of the grant of the patent: **02.03.2016**
(21) Application number: 11169558.1
(22) Date of filing: 10.06.2011
(51) Int. Cl.: A61M 5/168, A61M 5/145

(54) **A driving unit for a portable drug infusion device**
Antriebseinheit für tragbare Infusionsvorrichtung
Unité d'entrainement pour appareil d'infusion portable

(30) Priority: 11.06.2010 IT TO20100505
(43) Date of publication of application: 14.12.2011
(73) Proprietor: Cane' S.p.A., 10098 Rivoli (TO) (IT)
(72) Inventor: Cane', Mario, 10095 Collengo (TO) (IT)
(74) Representative: Robba, Pierpaolo

(56) References cited:
- WO-A2-2009/067502
- US-A- 3 631 847
- US-A- 3 701 345
- US-A- 4 747 828
- US-A- 5 808 203

## Description

The present invention relates to portable drug infusion devices and, more particularly, to a driving unit for such devices.

In the medical field, drug infusion devices, more commonly referred to as "infusion pumps", are often used for administering liquid drugs through a needle introduced into the patient's body, typically into a vein or under the skin.

Pumping devices used for infusion of substances into the human body are known for instance from WO 2009/067502, US 3,631,847 and US 3,701,345. WO 2009/067502 discloses a driving unit for a drug infusion device comprising the features disclosed in the preamble of claim 1. However, these devices are not compact and, on the contrary, they have a considerable size, since they are intended for use in hospital or laboratories, so that they are not suitable for being worn and carried by a patient.

As therapies requiring prolonged infusion of drugs are becoming increasingly diffused, even for patients who are not confined to bed, the need has arisen to provide compact, battery-powered infusion devices, which can be easily worn by the patient.

A known kind of portable infusion device comprises a housing or body on which a syringe containing a drug is removably mounted and inside which a seat for a driving unit is provided, said driving unit typically comprising an electric motor suitable for actuate a shaft to rotate and a mechanical assembly converting the rotary motion of said shaft into a linear motion that is transmitted to the syringe plunger, so as to obtain the drug infusion through a cannula connected to the needle inserted into the patient's body. The electric motor is controlled by a programmable electronic controller by which the desired infusion conditions can be set.

Italian patents IT 1257247 and IT 1261234, Italian utility model IT-U-193915, European patent EP 1078643 and European patent application EP 2174679, all in the name of the Applicant, disclose examples of these devices.

In these known devices, the mechanical assembly transmits the motion to a pusher associated to the syringe plunger by means of an axially sliding threaded rod that is partially housed inside the device body and penetrates inside the syringe during the infusion stroke.

This arrangement has some drawbacks.

First of all, it is required to protect the threaded rod from the syringe environment in order to prevent possible drug leakages from penetrating inside the housing and damaging the driving members. This is typically obtained by means of a bellows that is suitably fastened between the pusher and a base on which the syringe is mounted and surrounds the portion of the sliding threaded rod that penetrates into the syringe, thus completely separating the syringe environment from the inside of the housing. This makes the pump structure complex and more expensive. Furthermore, for the same external size, the means for fastening the bellows increase the pusher size and limit the plunger effective stroke, and thus the syringe capacity.

When the driving system is carried in the front portion of the body, it is also necessary to provide for thrust bearings for absorbing the backward thrust exerted on the rod by the drug coming out from the syringe and decreasing the corresponding friction between the contacting surfaces of the mechanical members actuating the pusher to move forward, said friction otherwise possibly causing the motor to block. This contributes to make the pump structure complex and more expensive. Moreover, in these cases the pressure sensors used for detecting possible occlusions in the drug path are annular sensors, which are not frequently used and also contribute to increase the pump costs.

EP 1219312, in the name of the Applicant, discloses a portable infusion pump wherein an externally threaded stem, integral in rotation with a toothed crown gear driven in rotation by the motor and housed inside the syringe plunger rod, meshes with the internal thread of a pusher associated to said rod. In this known pump the threaded stem does not penetrate inside the syringe body, whereby the problem of preventing possible drug leakages from penetrating inside the housing and damaging the driving members does not arise.

The object of the present invention is to provide a driving unit for an infusion pump allowing to overcome the above-mentioned drawbacks.

This object is achieved thanks to the fact that the driving unit is intended to drive a device wherein the syringe is mounted outside a body housing the driving unit itself, so that the rod, during its sliding motion, moves between the inside of the body and the inside of the syringe, and the rod is a hollow rod having a smooth external surface, receiving in its axial cavity an externally threaded stem, integral in rotation with a toothed crown gear driven in rotation by the motor, and having a rear end portion provided with an internal thread meshing with the external thread of said stem.

The internal thread is preferably obtained in a nut integral with the rod.

Advantageously, the rod slides in a guide having a non-circular cross-section and the nut has a non-circular external cross-section, having the same shape as the cross-section of said guide, so as to prevent said rod from rotating in the guide while sliding.

Alternatively, there is no guide and the nut is provided with a radial peg, the free end of which engages a groove formed in the internal surface of the body, in order to prevent the rod from rotating while sliding.

Some preferred embodiments if the invention, given by way of non-limiting example, will now be described with reference to the attached drawings, wherein:
- Fig. 1 is a view of a section taken along a vertical plane of a drug infusion device comprising a driving unit according to the invention;
- Fig. 2 is a view of a section taken along a plane passing through the line A-A of Fig. 1;
- Fig. 3 is a view of a horizontal section taken along a plane passing through the line B-B of Fig. 1;
- Fig. 4 is a perspective view of Fig. 1; and
- Fig. 5 is cross-sectional perspective view of the body of a device comprising a variant of the driving unit according to the invention.

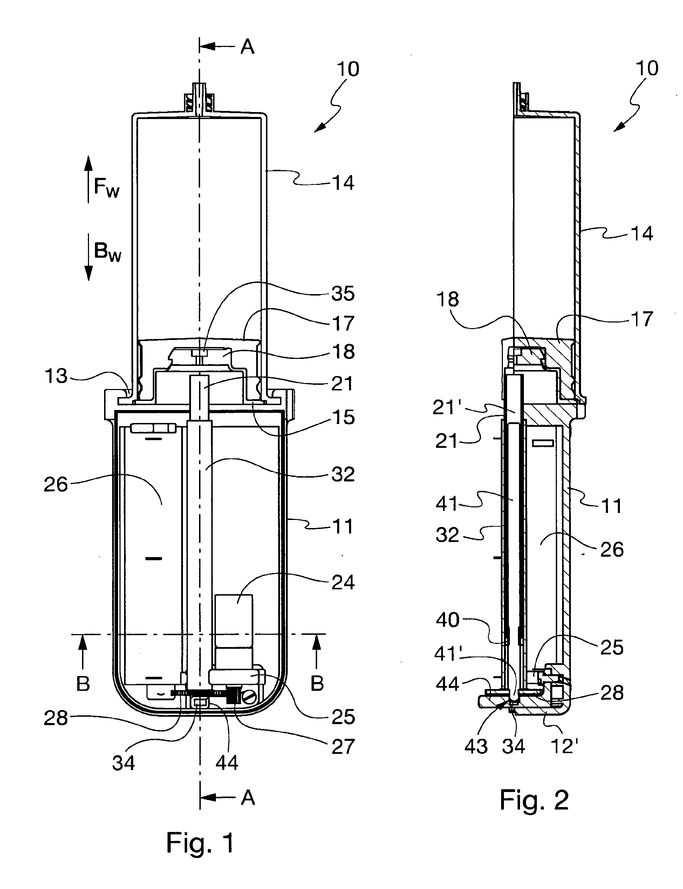

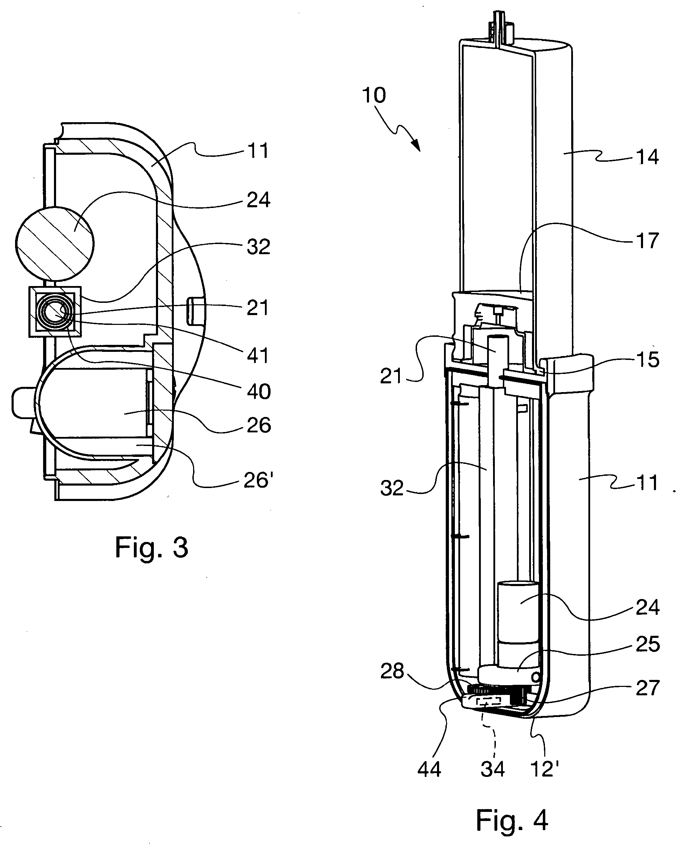

With reference to Figs. 1 - 4, a drug infusion pump 10 comprises a body or housing 11, having a compact size and intended to contain a driving unit according to the invention, which will be described later, and a syringe 14 suitable for containing a drug and connected to a needle inserted in the patient's body through a cannula, not shown. The syringe 14 is inserted by means of a bayonet coupling into a seat 13, externally formed in one of the bases of body 11, hereinafter referred to as front base 12, by means of a radial tab 15 engaging a slot in the wall of seat 13. Reference numeral 17 denotes the syringe plunger, associated with a pusher 18, which is axially movable to cause the forward or backward axial sliding of the plunger, depending on the operation phase.

The driving system includes a single electric motor 24 and a mechanical assembly, arranged to convert the rotary motion of motor 24 into a linear motion and to apply said linear motion to the pusher 18 of the syringe. The motor 24 is carried by a supporting ferrule 25 arranged in the rear portion of the body 11 and is controlled by a programmable electronic controller (not shown), also housed within the body 11. The motor and the controller are supplied by a battery 26, also housed within the body 11, in a seat 26'.

In order to receive the linear motion, pusher 18 is secured, for instance by means of a screw 35, to the front end of an axially slidable rod 21, which is partially received inside the body 11 and protrudes therefrom through an opening in the front base 12, penetrating, during its sliding motion, into the syringe internal cavity.

The rod 21 has a hollow body and a smooth external surface. An internally threaded nut 40 is fastened at the rear end of said rod, said nut meshing with an externally threaded stem 41, integral in rotation with a toothed crown gear 28, which in turn meshes with a pinion gear 27 carried by the shaft of the motor 24. The stem 41 is received in the axial cavity 21' of the rod 21. The nut 40 has a non-circular external cross-section, e.g. a square cross-section, and it slides together with the rod 21 in a correspondingly shaped guide 32. The rear end of the guide 32 is received in a correspondingly shaped seat provided in the ferrule 25, while its front end is secured to the internal face-of the front base 12.

The threaded stem 41 has a rounded rear end 41' abutting against the bottom of a seat 43 formed in a support member 44 carried by the rear base 12' of the body 11. Thanks to the rounded shape, the friction during the rotary motion can be reduced without the need for rolling-element bearings.

Said rear end 41' co-operates with a pressure sensor 34, connected to a respective signalling device arranged on the body 11, such as a LED or an acoustic signalling device (not shown), in order to detect an anomalous increase of the thrust applied to the rod 21 possibly caused by an occlusion in the flow path of the drug and to signal it to the patient.

During operation, the threaded stem 41, by rotating around its axis by effect of the rotary motion transmitted to the crown gear 28 by the pinion gear 27 integral with the shaft of the motor 24, imparts an axially sliding motion to the nut 40, thanks to the respective co-operating threads, and consequently to the rod 21 integral with said nut 40. Depending on the direction of the rotary motion of the motor 24, either a forward or a backward sliding motion of the rod 21, and consequently of the pusher 18 and of the plunger 17, is obtained, in the direction shown by the arrows Fw and, respectively, Bw (Fig. 1). The non-circular cross-sections of the rod 21 and of the guide 32 prevent the rod from rotating during the sliding motion.

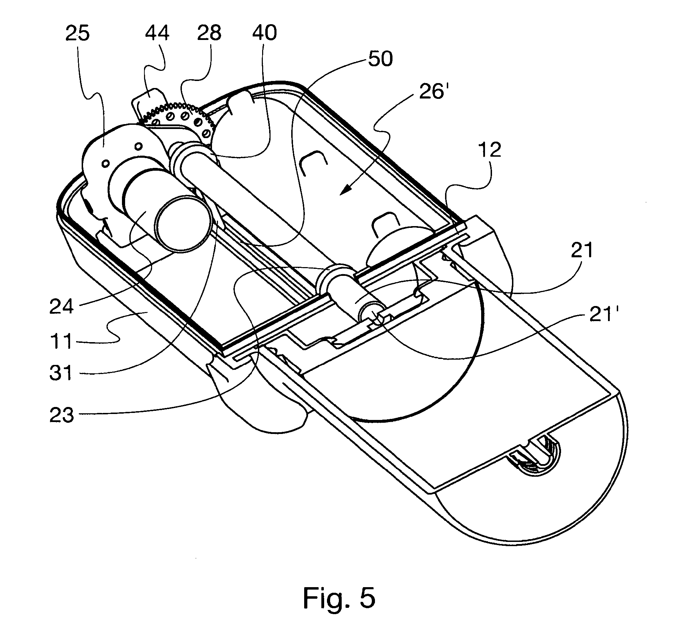

In the variant shown in Fig. 5, the square guide 32 is missing and the nut 40, of any shape, e.g. cylindrical, is externally provided with a radial peg 31, the free end of which engages a guiding groove 50 provided in the internal surface of the body 11, thus preventing the rod 21 from rotating. A guiding bushing 23 will be provided at the front base 12 of the body 11 for guiding the rod. Said bushing 23 can also define a corresponding sealing gasket for preventing dust and liquids, such as drug traces, from entering the body 11; to this aim, the bushing 23 can be made of rubber or similar material.

It is clear that the invention allows to achieve the intended objects. As there is no threaded part penetrating into the syringe body, the bellows for separating the internal environment of the syringe from the driving system is no longer required. By eliminating the bellows, a greater volume inside the syringe is available for the same external size, since the volume previously occupied by members for fastening the rear end of said bellow is available for increasing the stroke of the pusher and of the plunger.

Moreover, contrary to pumps wherein the driving members are supported in the front part of the body 11, no thrust bearing is required, since the backward thrust acting on the rod 21 by virtue of the resistance encountered by the liquid drug while coming out from the syringe is absorbed thanks to the rear end 41' of the stem 41 abutting against the support member 44. Furthermore, the pressure sensor can be a traditional disc-shaped sensor instead of an annular sensor.

It is evident that the above description is solely given by way of non-limiting example and that variants and modifications can be made without departing from the scope of the invention, as defined in the appended claims.

More particularly, even if in the shown embodiment the pinion gear 27 directly meshes with the crown gear 28, one or more reduction stages can be inserted between said pinion gear and said crown gear.

Furthermore, the stem 41 could mesh with a threaded portion of the internal surface of the rod 21 rather than with an internally threaded nut secured to the rear end of the thrust rod. It is clear that in this case in the embodiment of Figs. 1 - 4 at least a portion of the rod received in the guide 32 should have a non-circular cross-section.

## Claims

1. A driving unit for a portable drug infusion device (10), comprising an electric motor (24) operated by a battery (26) and controlled by a programmable electronic controller, and a mechanical assembly (21, 27, 28, 40) arranged to convert the rotary motion of the motor (24) into a linear motion and to impart said linear motion to the plunger (17) of a syringe (14), said mechanical assembly (21, 27, 28, 40) including a toothed crown gear (28) driven by a pinion gear (27) integral with a shaft of the motor (24), and an axially sliding rod (21) carrying, at a front end, a pusher (18) associated with the plunger (17), wherein said syringe (14) is mounted outside a body (11) housing the driving unit and said rod (21) moves, during its sliding motion, between the inside of the body (11) and the inside of the syringe (14), and wherein said rod (21) is a hollow rod having a smooth external surface and an axial cavity (21') housing an externally threaded stem (41) integral in rotation with said toothed crown gear (28), and having a rear end portion provided with an internal thread arranged to mesh with the external thread of said stem (41), **characterized in that** a rear- end (41') of said threaded stem (41) is rounded and abuts against the bottom of a seat (43) formed in a support member (44) associated with a rear base (12') of said body (11) and **in that** said rear end (41') cooperates with a pressure sensor (34), connected to a signalling device, for detecting and signalling to the patient an irregular increase in the thrust applied to said rod (21) because of a possible occlusion along the flow line of the drug from the syringe (14).

2. The driving unit as claimed in claim 1, **characterized in that** said internal thread is formed in an end portion of the internal surface of said hollow rod (21).

3. The driving unit as claimed in claim 1, **characterized in that** said hollow rod (21) is rigidly connected, at its rear end, with a nut (40) in which said internal thread is formed.

4. The driving unit as claimed in any of the preceding claims, **characterized in that** said rod (21) is slidable within a guide (32) having a non-circular cross-section, and said end portion of the rod (21) or said nut (40) has a non-circular cross-section, having the same shape as the cross-section of said guide (32), in order to prevent said rod (21) from rotating in the guide while sliding, a rear end of said guide (32) being received in a seat (42) of complementary shape, which is formed in a supporting ferrule (25) of the motor (24) arranged in the rear part of the body (11) and a front end of said guide being received in the internal face of a front base (12) of the body (11).

5. The device as claimed in any of the claims 1 to 3, **characterized in that** said end portion of the rod (21) or said nut (40) is provided with a radial peg (31), the free end of which engages a groove (50) formed on the internal surface of the body (11), in order to prevent said rod (21) from rotating while sliding.

6. The driving unit as claimed in claim 5, **characterized in that** said rod (21) is guided, at a front base (12) of the body (11), by a bushing (23) inserted in said base.

7. The driving unit as claimed in any of the preceding claims, **characterized in that** said signalling device comprises a LED or an acoustic signalling device.

8. The driving unit as claimed in claim 3, **characterized in that** said nut (40) in which the internal thread is formed has a non-circular cross-section and slides within a correspondingly shaped guide (32) along with the hollow rod (21).

9. The driving unit as claimed in claim 8, **characterized in that** said guide (32) has a square cross-section.

10. The driving unit as claimed in claim 6, **characterized in that** the guiding bushing (23) arranged at the front base (12) of the body (11) defines a corresponding sealing gasket for preventing dust and liquids from entering the body (11).

11. The driving unit as claimed in claim 10, **characterized in that** said bushing (23) is made of rubber or similar material.

12. A portable drug infusion device, **characterized in that** it comprises a driving unit according any of the claims 1 to 11.

13. The device according to claim 12, wherein said portable device is of the kind that can be easily worn and carried by the patient.

## Patentansprüche

1. Antriebseinheit für tragbare Infusionsvorrichtung (10), mit einem Elektromotor (24), der mit einer Batterie (26) betrieben und durch eine programmierbare elektronische Steuereinrichtung steuerbar ist, und mit einer mechanischen Anordnung (21, 27, 28, 40), die die Drehbewegung des Motors (24) in eine Linearbewegung umwandelt und die Linearbewegung auf den Kolben (17) einer Injektionspritze (14) überträgt, wobei die mechanische Anordnung (21, 27, 28, 40) ein gezahntes Tellerrad (28)aufweist, das von einem Ritzel (27) angetrieben ist, welches fest mit einer Welle des Motors (24) verbunden ist, und eine axial verschiebbare Stange (21) hat, die an einem vorderen Ende einen Schieber (18) trägt, der mit dem Kolben (17) verbunden ist, wobei die Injektionspritze (14) außerhalb eines Körpers (11) befestigt ist, in dem die Antriebseinheit untergebracht ist und sich die Stange (21) während ihrer Gleitbewegung zwischen der Innenseite des Körpers (11) und der Innenseite der Injektionspritze (14) bewegt, und wobei die Stange (21) eine hohle Stange ist, die eine glatte Außenfläche und einen axialen Hohlraum (21') aufweist, in dem ein Außengewindeschaft (41) untergebracht ist, der drehfest mit dem gezahnten Tellerrad (28) verbunden ist und einen hinteren Endabschnitt aufweist, der mit einem Innengewinde versehen ist, das mit dem Außengewinde des Schaftes (41) in Eingriff ist, **dadurch gekennzeichnet, dass** ein hinteres Ende (41') des Gewindeschaftes (41) abgerundet ist und sich am Boden eines Sitzes (43) in einem Trägerelement (44) abstützt, das mit einer hinteren Basis (12') des Körpers (11) verbunden ist, und dass das hintere Ende (41') mit einem Drucksensor (34) zusammenwirkt, der mit einer Signaleinrichtung verbunden ist, um einen auf die Stange (21) wirkenden unregelmäßigen Druckanstieg wegen einer möglichen Verstopfung längs des Strömungsflusses des Arzneimittels von der Injektionsspritze (14) zu erfassen und dem Patienten zu signalisieren,.

2. Antriebseinheit nach Anspruch 1, **dadurch gekennzeichnet, dass** das Innengewinde in einem Endabschnitt der Innenfläche der hohlen Stange (21) ausgebildet ist.

3. Antriebseinheit nach Anspruch 1, **dadurch gekennzeichnet, dass** die hohle Stange (21) an ihrem hinteren Ende mit einer das Innengewinde aufweisenden Mutter (40) starr verbunden ist.

4. Antriebseinheit nach einem der vorhergehenden Ansprüche, **dadurch gekennzeichnet, dass** die Stange (21) innerhalb einer Führung (32) mit einem nicht kreisförmigen Querschnitt verschiebbar ist und dass der Endabschnitt der Stange (21) oder die Mutter (40) einen nicht kreisförmigen Querschnitt mit der gleichen Form wie der Querschnitt der Führung (32) aufweist, um die Stange (21) während des Verschiebens an einer Drehung in der Führung zu hindern, wobei ein hinteres Ende der Führung (32) in einem Sitz (42) mit komplementärer Form aufgenommen ist, der in einem tragenden Klemmring (25) des von dem im hinteren Teil des Körpers (11) angeordneten Motor (24) vorgesehen ist, und ein vorderes Ende der Führung in der Innenfläche einer vorderen Basis (12) des Körpers (11) aufgenommen ist.

5. Vorrichtung nach einem der Ansprüche 1 bis 3, **dadurch gekennzeichnet, dass** der Endabschnitt der Stange (21) oder die Mutter (40) mit einem radialen Zapfen (31) versehen ist, dessen freies Ende in eine an der Innenfläche des Körpers (11) vorgesehene Nut (50) eingreift, um ein Drehen der Stange (21) während des Verschiebens zu verhindern.

6. Antriebseinheit nach Anspruch 5, **dadurch gekennzeichnet, dass** die Stange (21) an einer vorderen Basis (12) des Körpers (11) durch eine in die Basis eingesetzte Buchse (23) geführt ist.

7. Antriebseinheit nach einem der vorhergehenden Ansprüche, **dadurch gekennzeichnet, dass** die Signaleinrichtung eine LED oder eine akustische Signaleinrichtung aufweist.

8. Antriebseinheit nach Anspruch 3, **dadurch gekennzeichnet, dass** die Mutter (40), in der das Innengewinde vorgesehen ist, einen nichtkreisförmigen Querschnitt aufweist und innerhalb einer entsprechend geformten Führung (32) zusammen mit der hohlen Stange (21) gleitet.

9. Antriebseinheit nach Anspruch 8, **dadurch gekennzeichnet, dass** die Führung (32) quadratischen Querschnitt aufweist.

10. Antriebseinheit nach Anspruch 6, **dadurch gekennzeichnet, dass** die Führungsbuchse (23), die an der vorderen Basis (12) des Körpers (11) vorgesehen ist, eine entsprechende Dichtungsmanschette bildet, um zu verhindern, dass Staub und Flüssigkeit in den Körper (11) eindringen.

11. Antriebseinheit nach Anspruch 10, **dadurch gekennzeichnet, dass** die Buchse (23) aus Gummi oder einem ähnlichen Material hergestellt ist.

12. Tragbare Infusionsvorrichtung, **dadurch gekennzeichnet, dass** sie eine Antriebseinheit gemäß einem der Ansprüche 1 bis 11 aufweist.

13. Vorrichtung gemäß Anspruch 12, wobei die tragbare Vorrichtung von der Art ist, die von dem Patienten leicht getragen und mitgeführt werden kann.

## Revendications

1. Unité d'entraînement pour un dispositif portatif de perfusion de médicament (10), comprenant un moteur électrique (24) mis en oeuvre par une batterie (26) et commandé par un dispositif de commande électronique programmable, et un ensemble mécanique (21, 27, 28, 40) conçu pour convertir le mouvement de rotation du moteur (24) en un mouvement linéaire et pour communiquer ledit mouvement linéaire au piston (17) d'une seringue (14), ledit ensemble mécanique (21, 27, 28, 40) comprenant un engrenage à couronne dentée (28) entraîné par un pignon d'engrenage (27) d'un seul tenant avec un arbre du moteur (24), et une tige coulissant axialement (21) portant, à son extrémité avant, un pousseur (18) associé au piston (17), dans laquelle ladite seringue (14) est montée à l'extérieur d'un corps (11) logeant l'unité d'entraînement et ladite tige (21) se déplace, pendant son mouvement coulissant, entre l'intérieur dudit corps (11) et l'intérieur de ladite seringue (14), et dans laquelle ladite tige (21) est une tige creuse comportant une surface extérieure lisse et une cavité axiale (21') logeant une tige filetée extérieurement (41) tournant d'un seul tenant avec ledit engrenage à couronne dentée (28), et comportant une partie d'extrémité arrière dotée d'un filetage intérieur conçu pour venir en prise avec le filetage extérieur de ladite tige (41), **caractérisée en ce qu'**une extrémité arrière (41') de ladite tige filetée (41) est arrondie et vient en butée contre la partie inférieure d'un logement (43) formé dans un élément de support (44) associé à une base arrière (12') dudit corps (11) et **en ce que** ladite extrémité arrière (41') coopère avec un capteur de pression (34), connecté à un dispositif de signalisation, destiné à détecter une augmentation irrégulière de la poussée appliquée à ladite tige (21) en raison d'une possible occlusion de la conduite d'écoulement du médicament à partir de la seringue (14), ou à la signaler au patient.

2. Unité d'entraînement selon la revendication 1, **caractérisée en ce que** ledit filetage intérieur est formé dans une partie d'extrémité de la surface intérieure de ladite tige creuse (21).

3. Unité d'entraînement selon la revendication 1, **caractérisée en ce que** ladite tige creuse (21) est en prise fixe, au niveau de son extrémité arrière, avec un écrou (40) dans lequel est formé ledit filetage intérieur.

4. Unité d'entraînement selon l'une quelconque des revendications précédentes, **caractérisée en ce que** ladite tige (21) peut coulisser à l'intérieur d'un guide (32) ayant une section transversale autre que circulaire, et ladite partie d'extrémité de la tige (21) ou ledit écrou (40) a une section transversale autre que circulaire, ayant la même forme que la section transversale dudit guide (32), afin d'empêcher que ladite tige (21) ne tourne dans le guide tout en coulissant, une extrémité arrière dudit guide (32) étant reçue dans un logement (42) de forme complémentaire, qui est formé dans une ferrule de support (25) du moteur (24) disposée dans la partie arrière du corps (11), et une extrémité avant dudit guide étant reçue dans la face intérieure d'une base avant (12) du corps (11).

5. Dispositif selon l'une quelconque des revendications 1 à 3, **caractérisé en ce que** ladite partie d'extrémité de la tige (21) ou ledit écrou (40) est pourvu d'une clavette radiale (31), dont l'extrémité libre coopère avec une rainure (50) formée sur la surface intérieure du corps (11), afin d'empêcher que ladite tige (21) ne tourne en coulissant.

6. Unité d'entraînement selon la revendication 5, **caractérisée en ce que** ladite tige (21) est guidée, au niveau d'une base avant (12) du corps (11), par une douille (23) introduite dans ladite base.

7. Unité d'entraînement selon l'une quelconque des revendications précédentes, **caractérisée en ce que** ledit dispositif de signalisation comprend une DEL ou un dispositif de signalisation acoustique.

8. Unité d'entraînement selon la revendication 3, **caractérisée en ce que** ledit écrou (40) dans lequel est formé le filetage intérieur a une section transversale autre que circulaire et coulisse à l'intérieur d'un guide de forme correspondante (32) conjointement avec la tige creuse (21).

9. Unité d'entraînement selon la revendication 8, **caractérisée en ce que** ledit guide (32) a une section transversale carrée.

10. Unité d'entraînement selon la revendication 6, **caractérisée en ce que** la douille de guidage (23) disposée au niveau de la base avant (12) du corps (11) définit un joint d'étanchéité correspondant destiné à empêcher que de la poussière et des liquides ne pénètrent le corps (11).

11. Unité d'entraînement selon la revendication 10, **caractérisée en ce que** ladite douille (23) est constituée de caoutchouc ou d'un matériau similaire.

12. Dispositif portatif de perfusion de médicament, **caractérisé en ce qu'**il comprend une unité d'entraînement selon l'une quelconque des revendications 1 à 11.

13. Dispositif selon la revendication 12, dans lequel ledit dispositif portatif est du type pouvant être porté et transporté facilement par le patient.
